(19) Europäisches Patentamt
European Patent Office
Office européen des brevets

(11) EP 2 298 088 A1

(12) # EUROPÄISCHE PATENTANMELDUNG

(43) Veröffentlichungstag:
**23.03.2011 Patentblatt 2011/12**

(21) Anmeldenummer: **09169053.7**

(22) Anmeldetag: **31.08.2009**

(51) Int Cl.:
***A23L 3/32*** *(2006.01)* ***A23L 3/3454*** *(2006.01)*
***A23L 3/3463*** *(2006.01)* ***A23L 3/3481*** *(2006.01)*
***A23C 3/08*** *(2006.01)* ***A23L 2/44*** *(2006.01)*
***A23L 2/50*** *(2006.01)* ***A23C 3/07*** *(2006.01)*

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(71) Anmelder: **LANXESS Deutschland GmbH**
**51369 Leverkusen (DE)**

(72) Erfinder:
- **Läßker, Rebecca, Dipl.-Ing.**
  **53225 Bonn (DE)**
- **Ritzer, Edwin, Dr.**
  **51381 Leverkusen (DE)**

(54) **Verfahren zur Konservierung von Lebensmitteln**

(57) Verfahren zur Herstellung von mikrobiell stabilisierten Lebensmitteln **dadurch gekennzeichnet, dass** man ein Lebensmittel, enthaltend ein Dialkyldicarbonat mittels Elektroporation behandelt.

EP 2 298 088 A1

## Beschreibung

Die Erfindung betrifft ein Verfahren zur Herstellung von mikrobiell stabilisierten Lebensmitteln, insbesondere Getränken mittels Elektroporation.

Die Kaltentkeimung von Getränken ist in der Literatur vielfach beschrieben. Sie kann einerseits über chemische Zusätze wie Konservierungsmittel oder durch physikalische Verfahren wie thermische Sterilisation realisiert werden. Nachteile dieser Verfahren sind häufig unerwünschter Geschmack durch die Zusätze und/oder Zerstörung oder Veränderung von wichtigen Inhaltsstoffen beispielsweise durch die thermischen Verfahren.

Auf der Suche nach produktschonenden Verfahren ist in US-A-2005/0112251 die Kombination von Elektroporationsverfahren wie der Pulsed Electric Field (PEF) Methode in Kombination mit persistenten Fungiziden wie Natamycin oder Sorbat beschrieben.

Nachteilig bei diesem Verfahren ist allerdings, dass die persistenten Konservierungsmittel allen voran Sorbinsäure, in solch hohen Konzentrationen eingesetzt werden, dass sie in vielen Ländern die gesetzlichen Höchstmengen für spezielle Lebensmittel übersteigen. Trotz der hohen Einsatzmengen an Konservierungsmitteln wurde beim Schimmelpilz nur eine Sporeneinsaat von $10^2$ KBE/ mL (siehe Beispiel 2) und bei der vegetativen Hefe eine Keimeinsaat von nur $10^4$ KBE/ mL (siehe Beispiel 2) untersucht.

Aufgabe war es daher, ein Verfahren zur Stabilisierung von Lebensmitteln, insbesondere Getränken zu finden, das die beschriebenen Nachteile nicht aufweist und darüber hinaus nicht nur gegen Schimmelpilze und Hefen sondern auch gegen andere, vorzugsweise in Getränken vorkommenden Mikroorganismen eingesetzt werden kann.

Es wurde nun ein Verfahren zur Herstellung mikrobiell stabilisierter Lebensmittel, insbesondere Getränke gefunden, das dadurch gekennzeichnet ist, dass man ein Lebensmittel, enthaltend Dialkyldicarbonat mittels Elektroporation behandelt.

Besonders bevorzugt handelt es sich bei dem Dialkyldicarbonat um eine Verbindung der Formel (I)

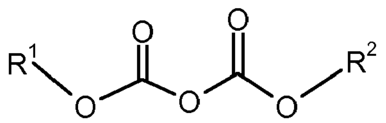 (I),

worin

$R^1$ und $R^2$ unabhängig voneinander für geradkettiges oder verzweigtes $C_1$-$C_8$-Alkyl, Cycloalkyl, $C_2$-$C_8$-Alkenyl, $C_2$-$C_8$-Alkinyl oder Benzyl stehen, welches jeweils gegebenenfalls ein- bis mehrfach, gleich oder verschieden substituiert ist durch Halogen, Nitro, Cyano, $C_1$-$C_6$-Alkoxy und/oder Dialkylamino, oder für Phenyl stehen, welches gegebenenfalls ein bis mehrfach, gleich oder verschieden substituiert ist durch Halogen, Nitro, Cyano, Alkyl, Halogenalkyl, Alkoxy, Halogenalkoxy, Acyl, Acyloxy, Alkoxycarbonyl und/oder Carboxyl,

bevorzugt

$R^1$ und $R^2$ unabhängig voneinander für geradkettiges oder verzweigtes $C_1$-$C_8$-Alkyl, $C_2$-$C_8$- Alkenyl oder Benzyl stehen,

besonders bevorzugt

$R^1$ und $R^2$ unabhängig voneinander für geradkettiges oder verzweigtes $C_1$-$C_5$-Alkyl, $C_3$-Alkenyl oder Benzyl stehen,

und ganz besonders bevorzugt

$R^1$ und $R^2$ unabhängig voneinander für Methyl, Ethyl, Isopropyl, tert.-Butyl, tert.-Amyl, Allyl oder Benzyl stehen.

Ganz besonders bevorzugt ist als Dialkyldicarbonat Dimethyldicarbonat (DMDC).

Das Dialkyldicarbonat wird vorzugsweise in einer Menge von 1 bis 300 ppm, insbesondere von 10 bis 260 ppm, bezogen auf das Lebensmittel, insbesondere Getränk zugesetzt.

Die Substanzklasse der Dialkyldicarbonate hat die besondere Eigenschaft, in Kontakt mit entsprechenden (wässrigen) Lebensmitteln, insbesondere Getränken in die abgeleiteten Alkohole und Kohlendioxid zu hydrolysieren. In

Abhängigkeit von der Temperatur der Getränke während der Anwendung ist deshalb schon nach relativ kurzer Zeit die eigentlich aktive Substanz nicht mehr im Getränk vorhanden. Bei den üblichen Temperaturen der Kaltabfüllung von Getränken von 0 bis 25 °C ist dies nach einigen Stunden der Fall. Die Wirkung von Dialkyldicarbonaten durch verschiedene Methoden oder Kombinationen zu verbessern ist bereits in der Patentliteratur vielfach beschrieben beispielsweis in DE-A-4434314, US-A-5738888, WO 200187096 oder US-A-2001046538.

Bei der Erfindung handelt es sich vorzugsweise um ein bei Raumtemperatur pumpbares Lebensmittel. Unter Lebensmittel werden im Rahmen der Erfindung Stoffe oder Erzeugnisse verstanden, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden. Zu "Lebensmitteln" zählen auch Getränke, Kaugummi sowie alle Stoffe - einschließlich Wasser -, die dem Lebensmittel bei seiner Herstellung oder Ver- oder Bearbeitung absichtlich zugesetzt werden.

Besonders bevorzugte Lebensmittel sind Getränke, insbesondere Tee-basierte Getränke, einschließlich grünem Tee, schwarzem Tee sowie anderen Tee-Sorten, sowie um sauer eingestellte Getränke, insbesondere mit einem pH ≤ 4.2, karbonisierte und nicht-carbonisierte alkoholfreie Erfrischungsgetränke, Fruchtsäfte, Fruchtnektare, Fruchsafthaltige Getränke, Frucht-zubereitungen, Weine, alkoholfreie Getränke, Ciders, Eistees, alkoholische Mischgetränke, aromatisierte Wässer oder Sportgetränke bzw. isotonische Getränke.

Weiterhin bevorzugt ist es, ggf. weitere antimikrobiell wirkende Stoffe, insbesondere persistente Konservierungsmittel mit zu verwenden.

Es ist daher bevorzugt, dass dem zu behandelnden Lebensmittel, insbesondere Getränk weitere dieser Stoffe, insbesondere zusätzlich wenigstens ein weiteres antimikrobiell wirkendes Konservierungsmittel aus der Gruppe der polyenen Antimykotika, wie beispielsweise Natamycin, Nystatin, Lucensomycin oder Amphotericin B, organischen Säuren, wie beispielsweise Benzoesäure, Sorbinsäure, Propionsäure, oder Milchsäure, Salze der genannten Säuren, wie beispielsweise Benzoate, Sorbate, Propionate oder Laktate Imidazole oder deren Salze, insbesondere Imazalil, Schwefeldioxid, EDTA und Lysozym zugegeben werden.

Besonders bevorzugt sind Natriumbenzoat und Kaliumsorbat.

Vorzugsweise enthält das nach dem erfindungsgemäßen Verfahren zu behandelnde Lebensmittel, insbesondere Getränk wenigstens ein weiteres antimikrobiell wirkendes Konservierungsmittel, insbesondere eines aus der oben genannten Gruppe.

Im Falle einer Mitverwendung von weiteren antimikrobiell wirkenden Stoffen werden diese vorzugsweise in einer Menge von (bei Salzen bezogen auf die freie Säure) 1 bis 2000 ppm, insbesondere von 25 bis 500 ppm, bezogen auf das Lebensmittel, insbesondere Getränk eingesetzt.

Die Zugabe der Dialkyldicarbonate, insbesondere von Dimethyldicarbonat erfolgt üblicherweise in flüssiger Form, portionsweise oder kontinuierlich zu dem Lebensmittel, insbesondere Getränk. Bevorzugt ist es, dass die Zugabe von Dialkyldicarbonat vorzugsweise kontinuierlich mittels einer Düse, insbesondere mit einem Druck von 0,1 bis 40bar, vorzugsweise von 0,5 bis 40 bar, insbesondere 10 bis 35 bar gegenüber dem Getränkedruck erfolgt. Insbesondere DMDC wird bevorzugt mittels einer Dosierpumpe über eine beheizte Düse, in den Getränkestrom verdüst. Verbesserungen entsprechender Pumpen wurden in der Patentliteratur beschrieben beispielsweise in der DE-A-2910328 oder in der DE-A-2930765. Verbesserungen der Düse bzw. der davor liegenden Mischkammer wurden beispielsweise in der DE-A-1557043 beschrieben. Entsprechende Dosiervorrichtungen bestehen üblicherweise aus Vorratsgefäßen, elektromagnetisch betriebener Dosierpumpe, Eindüsungsbereich und einem elektronisch gekoppelten induktiven Durchflussmessgerät, sowie selbstverständlich Ansaugvorrichtungen, Belüftung bzw. Temperierung, Verbindungen, Ventilen, Sensoren etc. samt verbindenden und steuernden Elektronikelementen. Die Dosierleistungen der Pumpen betragen üblicherweise 0,1 bis 20 Liter DMDC pro Stunde.

Die Zugabe von Dialkyldicarbonat, insbesondere DMDC zum Lebensmittel, insbesondere Getränk erfolgt vorzugsweise bei einer Temperatur von -5 bis 30°C, insbesondere bei 0 bis 25°C, besonders bevorzugt bei 5 bis 22°C.

Bei Mitverwendung von weiteren antimikrobiell wirkenden Stoffen kann diese entweder getrennt oder gemeinsam mit dem Dimethyldicarbonat zum Getränk erfolgen.

Bevorzugt erfolgt nach der Zugabe des anderen antimikrobiell wirkenden Stoffes die Zugabe der Dialkyldicarbonatverbindung.

Aufgrund des Zerfalls der Dialkyldicarbonatverbindung im Lebensmittel, insbesondere Getränk ist es bevorzugt, nach der Zugabe der Dialkyldicarbonatverbindung die Elektroporation möglichst kurz danach anzuschließen. So erfolgt bevorzugt weniger als 15 Minuten nach der Dialkyldicarbonat-Zugabe, vorzugsweise nach weniger als 5 Minuten, die Elektroporation. Die Elektroporation, insbesondere das als PEF (pulsed electric field) bekannte Verfahren, das auch als high intensity pulsed electric field bezeichnet wird, im Sinne der Erfindung ist ein Verfahren, das vorzugsweise dadurch gekennzeichnet ist, dass man gepulste elektrischer Felder auf das Lebensmittel einwirken lässt. Verfahrensparameter sind primär die elektrische Feldstärke und der Elektroenergieeintrag.

Bei dem Elektroporations-Verfahren PEF werden Lebensmittel mit Hochspannungspulsen vorzugsweise mit Feldstärken von 0,5 bis 100 kV/cm zwischen zwei Elektroden behandelt. Das PEF-Verfahren wird vorzugsweise bei -10

bis 60 °C, insbesondere bei 15 bis 25°C durchgeführt. Das Lebensmittel ist dabei vorzugsweise für weniger als 1 s der Energie ausgesetzt, wobei die Erhitzung des Lebensmittels minimiert wird. Im Gegensatz zu den thermischen Verfahren wird die PEF- Technologie als besseres Verfahren betrachtet, da hierdurch die sensorischen und physikalischen Eigenschaften von Lebensmitteln nicht oder nur kaum beeinflusst werden Die hohe Feldstärke bei der PEF Technologie wird im allgemeinen dadurch erreicht, dass ein großer Teil der Energie in einer Kondensatorbank eines Gleichstromnetzteils gespeichert wird, welches sich dann in Form von Hochspannungspulsen entlädt.

Beispiele solcher PEF-Geräte, bzw. der Beschreibung ihrer Funktionsweise können beispielsweise der DE-A-3413583 entnommen werden.

Bei der Elektroporation, insbesondere dem PEF-Verfahren kommen erfindungsgemäß vorzugsweise Energiedichten von 1 bis 1000 J/ml Lebensmittel, insbesondere Getränk zum Einsatz, vorzugsweise 15 bis 200 J/ml. Für das erfindungsgemäße Verfahren sind Feldstärken von vorzugsweise 0,5 bis 100 kV/cm, insbesondere 3 bis 50 kV/cm vorteilhaft. Die Frequenz der elektrischen Impulse beträgt vorzugsweise 10 bis 800 Hz, vorzugsweise 60 bis 500 Hz.

Die Dauer der Pulse (Pulsbreite) beträgt vorzugsweise 1 bis 100 µs, insbesondere 5 bis 50 µs.

Die Energiedichte ist dabei wie folgt definiert.

$$Energiedichte = \frac{3600 * Wirkungsgrad * aufgenommeneLeistung[W]}{Durchfluss[l/h] * 1000}$$

Der Wirkungsgrad beträgt beispielsweise bei dem in den Beispielen eingesetzten Elcrack®-Gerät ca. 85 %. Die aufgenommene Leistung in Watt wurde während des Behandlungsverfahrens vom PEF-Gerät abgelesen. Der Durchfluss betrug 70 1/h.

Das behandelte Lebensmittel, insbesondere Getränk wird in der Regel in Abhängigkeit von dem Elektroenergieeintrag um ca. 1 bis 20°C erwärmt.

Besonders bevorzugt wird das erfindungsgemäße Verfahren gegen folgende Stämme eingesetzt: Bakterien (z. B. *Bacillus* spp., *Lactobacillus* spp., *Leuconostoc* spp., *Acetobacter* spp., *Gluconacetobacter* spp., *Alicyclobacillus* spp.), Hefen (z. B. *Saccharomyces* spp., *Zygosaccharomyces* spp., *Trichoderma* spp., *Candida* spp., *Brettanomyces* spp., *Pichia* spp.) und Schimmel (z. B. *Penicillium* spp., *Byssochlamys* spp., *Aspergillus* spp., *Fusarium* spp.). Überraschenderweise wurde gefunden, dass das erfindungsgemäße Verfahren zu einer deutlichen Verbesserung der Entkeimung führt, was zur Folge hat, dass entweder höhere Anfangsverkeimungen von Getränken mit gleichen Mengen an Dialkyldicarbonat ggf. in Kombination mit persistenten Konservierungsmitteln bekämpft werden können oder bei normaler Verkeimung geringe Mengen bereits ausreichen.

Das erfindungsgemäße Verfahren hat somit den Vorteil Mikroorganismen effektiv zu bekämpfen, ohne bzw. ohne hohe und ggf. gar nicht zugelassene Mengen an persistenten Konservierungsmitteln auszukommen.

Zusätzlich wurde gefunden, dass Bakteriensporen und Bakterien, die Schleimbildner sind und somit in der Lage, Biofilme zu produzieren, welche große Probleme in der Lebensmittelindustrie darstellen (z.B. *Lactobacillus frigidus*) effektiv bekämpft werden können. Außerdem wurden endosporenbildende Bakterien am Beispiel von *Bacillus subtilis* untersucht. Endosporen sind Überdauerungsformen von Bakterien (z. B. *Bacillus, Clostridium),* die im Vergleich zu vegetativen Bakterienzellen in der Regel 100°C mehrere Stunden überdauern können und erst nach einer mehrere Minuten langen Erhitzung auf 120°C sicher abgetötet werden. Auch gegen diese Mikroorganismen konnte das erfindungsgemäße Verfahren erfolgreich eingesetzt werden.

**<u>Beispiele:</u>**

In den folgenden Beispielen wurde für die PEF-Behandlung das Gerät Elcrack® des Deutschen Instituts für Lebensmitteltechnik e. V. eingesetzt.

Die Behandlungszelle des Gerätes hatte dabei einen Durchmesser von 5 mm und einen Elektrodenabstand von 7 mm. Die Behandlung erfolgte bei unterschiedlichen Feldstärken und Energiedichten, wobei die Pulsbreite 20 µs und die Pulsfrequenz 400 Hz betrugen.

In den nachfolgenden Beispielen wurde jeweils klarem Apfelsaft unter Rühren mittels eines Propellerrührers eine Keim- bzw. Sporensuspension, dann nach ca. 3 Minuten ggf. weitere antimikrobiell wirksamen Stoffe und danach nach ca. 2 Minuten DMDC. Die Keim- bzw. Sporensuspenison wurde so zugesetzt, dass sich im Apfelsaft eine bestimmte Menge an Zellen in der Regel $10^3$ bis $10^5$ KBE/ mL befanden.

Der Apfelsaft wurde danach PEF behandelt (mit dem oben beschriebenen Elcrack® Gerät). Als weitere mikrobiell wirksame Stoffe kamen Natriumbenzoat bzw. Kaliumsorbat zum Einsatz.

Bei unterschiedlichen Energiedichten und Feldstärken wurden die in der Tabelle angegebenen Temperaturen

der endbehandelten Getränke festgestellt.

Als Maß für die Keimreduzierung wurde die mittlere logarithmische Keimreduzierung (MLK) bestimmt. Hierbei wird der Logarithmus der Überlebendkeimzahl vom Logarithmus der Anfangskeimzahl subtrahiert. Je höher der MLK-Wert, desto höher die Keimreduzierung und desto besser die Wirkung.

V steht für Vergleichsbeispiel

Die verwendeten Mikroorganismen waren die folgenden.

A: *Bacillilus subtilis* DSM 347 (ATCC 6633)

B: *Penicillium roqueforti* DSM 1079 (ATCC 34908)

C: *Lactobacillus frigidus* DSM 6235

D: *Saccharomyces cervisiae* DSM 70449 (ATCC 18824)

**Tabelle:**

| Bsp. | Mikroorganismus | [KBE/ mL] | Antimikrobiell wirkender Stoff/ Menge in ppm 1) | Feldstärke [kV/ cm] | Energiedichte [J/ mL] | Temperatur nach Behandlung | MLK/ Zeit 2) |
|---|---|---|---|---|---|---|---|
| A1 | *Bacillus subtilis* | $10^3$ | DMDC/ 250 | 35 | 111 | 36°C | 2.2/ 24 h |
| A2 | " | $10^3$ | DMDC/250 Benzoat/ 150 | 35 | 110 | 36°C | 3.0/24 h |
| A3 | " | $10^3$ | DMDC/ 250 Sorbat/ 300 | 35 | 116 | 36°C | 2.1/ 24 h |
| A4 | " | $10^3$ | DMDC/ 250 | 15 | 23 | 25°C | 1.4/ 24 h |
| A5 | " | $10^3$ | DMDC/ 250 | 35 | 111 | 36°C | 3,0/ 1 Woche |
| VA1 | " | $10^3$ | Benzoat/ 150 | 35 | 111 | 36°C | 2.0/ 24 h |
| VA2 | " | $10^3$ | Sorbat/ 300 | 35 | 116 | 36°C | 0.5/ 24 h |
| VA3 | " | $10^3$ | -/- | 35 | 111 | 36°C | 2.7/ 1 Woche |
| VA4 | " | $10^3$ | - / - | - | - | 21°C | Keine; total verkeimt |
| B 1 | *Penicillium roqueforti* | $10^5$ | DMDC/ 250 | 30 | 94 | 32°C | mind. 7/ 24 h |
| B2 | " | $10^5$ | DMDC/ 250 Benzoat/ 150 | 30 | 103 | 32°C | mind. 7/ 24 h |
| B3 | " | $10^5$ | DMDC/ 250 Sorbat/ 300 | 30 | 104 | 32°C | mind. 7/ 24 h |
| B4 | " | | " DMDC/ 250 Sorbat/ 300 | 30 | 104 | 32°C | mind. 7/ 4 Wochen |
| B5 | " | | " DMDC/ 250 | 30 | 94 | 32°C | mind. 7/ 4 Wochen |
| VB1 | " | $10^5$ | DMDC/ 250 | - | - | 21°C | 6.4/ 24 h |
| VB2 | " | $10^5$ | -/- | 30 | 98 | 32°C | Kein; großes Keimwachstum/ 24 h |
| VB3 | " | | " Benzoat/ 150 | 30 | 99 | 32°C | " |
| VB4 | " | " Sorbat/ 300 | | 30 | 104 | 32°C | " |

(fortgesetzt)

| Bsp. | Mikroorganismus | [KBE/ mL] | Antimikrobiell wirkender Stoff/ Menge in ppm 1) | Feldstärke [kV/ cm] | Energiedichte [J/ mL] | Temperatur nach Behandlung | MLK/ Zeit 2) |
|---|---|---|---|---|---|---|---|
| VB5 | *Penicillium roqueforti* | $10^5$ | -/- | 30 | 98 | 32°C | Keine; großes Keimwachstum/ 4 Wochen |
| C1 | *Lactobacillus frigidus* | $10^5$ | DMDC/ 250 | 30 | 93 | 32°C | 3.6/ 1 Woche |
| C2 | " | " DMDC/ | 250 Benzoat/ 150 | 30 | 92 | 32°C | 3.5/ 1 Woche |
| C3 | " | | " DMDC/ 250 Sorbat/ 300 | 30 | 91 | 32°C | 3.6/ 1 Woche |
| VC1 | " | | " DMDC/ 250 | - | - | 21°C | 2.3 / 1 Woche |
| VC2 | " | | " Benzoat/ 150 | 30 | 88 | 32°C | 2.8/ 1 Woche |
| VC3 | " | "Sorbat/ 300 | | 30 | 91 | 32°C | 3.0/ 1 Woche |
| VC4 | " | " | -/- | 30 | 83 | 32°C | 3.4/ 1Woche |
| VC5 | " | " -/- | | - | - | 21°C | 2.3/ 1Woche |
| D1 | *Saccharomyces cerivisiae* | $10^5$ | DMDC/ 250 | 5 | 3 | 22°C | min 7/ 24 h |
| D2 | " | | " DMDC/ 250 Benzoat/ 150 | 5 | 3 | 22°C | min 7/ 24 h |
| D3 | " | | " DMDC/ 250 Sorbat/ 300 | 5 | 3 | 22°C | min 7/ 24 h |
| VD1 | " | " | -/- | 5 | 2 | 22°C | Keine; großes Keimwachstum/ 24 h |
| VD2 | " | | " Benzoat/ 150 | 5 | 3 | 22°C | " |
| VD3 | " | " Sorbat/300 | | 5 | 6 | 22°C | " |

1) Menge Sorbat und Benzoat sind jeweils als freie Säure angegeben
2) Für den Wert "mind. 7" gilt: Die Keimeinsaat bei den vegetativen Zellen betrug$10^5$ KBE/ mL. Für die mikrobiologischen Untersuchungen wurden 100 mL der Probe membranfiltriert und analysiert, weshalb sich dann ein Wert von mind. 7 ergibt, da nichts gewachsen war.

## Patentansprüche

1. Verfahren zur Herstellung von mikrobiell stabilisierten Lebensmitteln **dadurch gekennzeichnet, dass** man ein Lebensmitteln, enthaltend ein Dialkyldicarbonat mittels Elektroporation behandelt.

2. Verfahren gemäß Anspruch 1, **dadurch gekennzeichnet, dass** es sich bei dem Dialkyldicarbonat um eine Verbindung der Formel (I) handelt

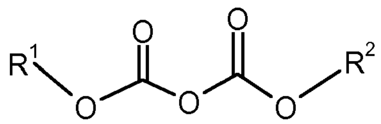

(I)

worin

$R^1$ und $R^2$ unabhängig voneinander für geradkettiges oder verzweigtes $C_1$-$C_8$-Alkyl, Cycloalkyl, $C_2$-$C_8$-Alkenyl, $C_2$-$C_8$-Alkinyl oder Benzyl stehen, welches jeweils gegebenenfalls ein- bis mehrfach, gleich oder verschieden substituiert ist durch Halogen; Nitro; Cyano; $C_1$-$C_6$-Alkoxy; Dialkylamino; oder für Phenyl stehen, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl,

bevorzugt

$R^1$ und $R^2$ unabhängig voneinander für geradkettiges oder verzweigtes $C_1$-$C_8$-Alkyl, $C_2$- $C_8$-Alkenyl oder Benzyl stehen,

besonders bevorzugt

$R^1$ und $R^2$ unabhängig voneinander für geradkettiges oder verzweigtes $C_1$-$C_5$-Alkyl, $C_3$-Alkenyl oder Benzyl stehen,

und ganz besonders bevorzugt

$R^1$ und $R^2$ unabhängig voneinander für Methyl, Ethyl, Isopropyl, tert.-Butyl, tert.- Amyl, Allyl oder Benzyl stehen.

3. Verfahren gemäß Anspruch 1, **dadurch gekennzeichnet, dass** es sich bei dem Dialkyldicarbonat um Dimethyldicarbonat handelt.

4. Verfahren gemäß Anspruch 1, **dadurch gekennzeichnet, dass** es sich bei dem Lebensmittel um ein Getränk handelt.

5. Verfahren gemäß Anspruch 1, **dadurch gekennzeichnet, dass** es sich bei dem Lebensmittel um Tee-basierte Getränke, einschließlich grünem Tee, schwarzem Tee sowie anderen Tee-Sorten, sowie um sauer eingestellte Getränke mit einem pH $\leq 4.2$, karbonisierte und nicht-carbonisierte alkoholfreie Erfrischungsgetränke, Fruchtsäfte, Fruchtnektare, Fruchsaft-haltige Getränke, Fruchtzubereitungen, Weine, alkoholfreie Getränke, Ciders, Eistees, alkoholische Mischgetränke, aromatisierte Wässer, Sportgetränke oder isotonische Getränke handelt.

6. Verfahren gemäß Anspruch 1, **dadurch gekennzeichnet, dass** dem zu behandelnden Lebensmittel zusätzlich ein weiteres antimikrobiell wirkendes Konservierungsmittel zugegeben wird.

7. Verfahren gemäß Anspruch 1, **dadurch gekennzeichnet, dass** dem zu behandelnden Lebensmittel zusätzlich wenigstens ein weiteres antimikrobiell wirkendes Konservierungsmittel aus der Gruppe Benzoesäure, Benzoate, Sorbinsäure, Sorbate, Propionsäure, Propionate, Natamycin, Nisin, Schwefeldioxid, EDTA und Lysozym zugegeben wird.

8. Verfahren gemäß Anspruch 1, **dadurch gekennzeichnet, dass** die mittels Elektroporation ins Lebensmittel Getränk eingebrachte Energiedichte 1 bis 1000 J/ml beträgt.

**9.** Verfahren gemäß Anspruch 1, **dadurch gekennzeichnet, dass** als Elektroporation das pulsed electric field-Verfahren eingesetzt wird.

**10.** Verfahren gemäß Anspruch 1, **dadurch gekennzeichnet, dass** das Dialkyldicarbonat in einer Menge von 10 bis 250 ppm, bezogen auf das Lebensmittel, insbesondere Getränk zugesetzt wird.

Europäisches Patentamt
European Patent Office
Office européen des brevets

# EUROPÄISCHER RECHERCHENBERICHT

Nummer der Anmeldung
EP 09 16 9053

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,Y | WO 03/070026 A (DSM IP ASSETS BV [NL]; STARK JACOBUS [NL]; DUTREUX NICOLE LILIANE [NL]) 28. August 2003 (2003-08-28)<br>* Seiten 1-6; Ansprüche 4,6,10,14-17; Beispiele 4,7,10 * | 1-10 | INV.<br>A23L3/32<br>A23L3/3454<br>A23L3/3463<br>A23L3/3481<br>A23C3/08<br>A23L2/44<br>A23L2/50<br>A23C3/07 |
| Y | US 2008/311259 A1 (SINGH PREM S [US] ET AL) 18. Dezember 2008 (2008-12-18)<br>* Absätze [0009], [0011], [0025] - [0027]; Ansprüche 10,13 * | 1-10 | |
| D,Y | US 6 136 356 A (BUNGER JOHN ROBERT [US] ET AL) 24. Oktober 2000 (2000-10-24)<br>* Spalten 4-5; Ansprüche; Beispiele * | 1-10 | |
| D,Y | US 5 738 888 A (CIRIGLIANO MICHAEL CHARLES [US] ET AL) 14. April 1998 (1998-04-14)<br>* Spalten 2-4; Ansprüche 1,2,10,11 * | 1-10 | |
| Y | US 6 803 064 B1 (GIVEN PETER [US] ET AL) 12. Oktober 2004 (2004-10-12)<br>* Spalte 4, Zeile 33 - Spalte 5, Zeile 6; Ansprüche 8,9,14,15 * | 1-10 | RECHERCHIERTE SACHGEBIETE (IPC) |
| A | GALVEZ A; ABRIOUEL H; LUCAS-LOPEZ R; BEN OMRAR N.: "Bacteriocin-based strategies for food biopreservation"<br>INTERNATIONAL JOURNAL OF MICROBIOLOGY, 2007, Seiten 51-70, XP002556769<br>item 3.4<br>* Seiten 58-60 * | 1-10 | A23L<br>A23C |

Der vorliegende Recherchenbericht wurde für alle Patentansprüche erstellt

| Recherchenort | Abschlußdatum der Recherche | Prüfer |
|---|---|---|
| München | 23. November 2009 | Muller, Isabelle |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 09 16 9053

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-11-2009

| Im Recherchenbericht angeführtes Patentdokument | | Datum der Veröffentlichung | Mitglied(er) der Patentfamilie | | | Datum der Veröffentlichung |
|---|---|---|---|---|---|---|
| WO 03070026 | A | 28-08-2003 | AU | 2003210351 | A1 | 09-09-2003 |
| | | | BR | 0307583 | A | 01-02-2005 |
| | | | US | 2005112251 | A1 | 26-05-2005 |
| | | | ZA | 200405946 | A | 29-09-2005 |
| US 2008311259 | A1 | 18-12-2008 | KEINE | | | |
| US 6136356 | A | 24-10-2000 | AU | 6512898 | A | 24-11-1998 |
| | | | BR | 9809000 | A | 08-08-2000 |
| | | | CA | 2285462 | A1 | 05-11-1998 |
| | | | CN | 1257409 | A | 21-06-2000 |
| | | | EP | 0977499 | A1 | 09-02-2000 |
| | | | WO | 9848649 | A1 | 05-11-1998 |
| | | | JP | 2001523107 | T | 20-11-2001 |
| | | | US | 2001046538 | A1 | 29-11-2001 |
| | | | US | 6132787 | A | 17-10-2000 |
| US 5738888 | A | 14-04-1998 | KEINE | | | |
| US 6803064 | B1 | 12-10-2004 | KEINE | | | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20050112251 A
- DE 4434314 A
- US 5738888 A
- WO 200187096 A
- US 2001046538 A
- DE 2910328 A
- DE 2930765 A
- DE 1557043 A
- DE 3413583 A